# EUROPEAN PATENT SPECIFICATION

(11) **EP 2 346 485 B1**
(45) Date of publication and mention of the grant of the patent: **02.08.2017**
(21) Application number: 09748361.4
(22) Date of filing: 15.10.2009
(51) Int. Cl.: A61K 8/44, A61Q 5/02, A61Q 5/12, A61Q 19/00

(54) **USE OF BETAINE**
VERWENDUNG VON BETAIN
UTILISATION DE BETAÏNE

(30) Priority: 15.10.2008 FI 20085971; 15.10.2008 US 105620 P
(43) Date of publication of application: 27.07.2011
(73) Proprietor: DuPont Nutrition Biosciences ApS, 1411 Copenhagen K (DK)
(72) Inventor: JUTILA, Kirsti, FI-02170 Espoo (FI)
(74) Representative: D Young & Co LLP
(86) International application number: PCT/FI2009/050830
(87) International publication number: WO 2010/043769

(56) References cited:
- WO-A1-01/39730
- WO-A1-91/18588
- WO-A1-97/48371
- WO-A2-2004/024106
- WO-A2-2006/103056
- DE-A1- 10 133 201
- FR-A1- 2 877 843
- JP-A- 10 182 409
- JP-A- 2003 095 956
- US-A1- 2002 136 787
- RIGANO L DELL'ACQUA G LEPORATTI R: "Benefits of trimethylglycine (Betaine) in personal-care formulations", COSMETICS & TOILETRIES, WHEATON, IL, US, vol. 115, no. 12, 1 December 2000 (2000-12-01), pages 47-53, XP002954567, ISSN: 0361-4387

## Description

### Field of the invention

The present invention relates to use of betaine for protecting skin from biological irritation. The invention also relates to the use of betaine as protecting agent in a cosmetic and/or toiletry preparation. The invention further relates to a method of protecting skin from biological irritation by sweat or sebum.

### Background of the invention

Skin is prone to several types of irritation, such as mechanical, thermal, chemical and/or biological irritation.

Patent publication EP 531 387 B discloses that betaine is effective in neutralizing the skin-irritating properties of a cosmetic composition. Publication EP 930 870 B describes that betaine protects skin from mechanical irritation, such as scratching, abrading and cutting.

In addition to these publications, Rigano, L., et al. (2000) Cosmetics & Toiletries magazine Vol. 115, No.12, p. 47-54 describe generally the benefits of betaine in personal care formulations in the cosmetic's field.

Patent publication US 6,660,304 describes a method for treating *Miliaria rubra,* commonly known as prickly heat, by topically applying a composition comprising betaine and *Scutellaria baicalensis* extract. *Miliaria rubra* is a skin disease or disorder, clinical reaction consisting of red, itchy or stingingly blistering pimples. Typically, the lesions are found in the skin of the body area.

It does not appear from the above-mentioned publications that betaine protects skin from biological irritation by sweat or sebum.

### Brief description of the invention

The present invention provides betaine for use as a medicament in protecting skin from irritation induced by a compound secreted by a mammalian body through the skin; wherein said compound is selected from sweat, sebum and individual ingredients or mixtures of ingredients included in the secretory products.

Described herein is the use of betaine for protecting skin from biological irritation by sweat or sebum. Further described herein is the use of betaine for protecting scalp from biological irritation by sweat or sebum. Also described herein is a method of protecting skin from biological irritation comprising applying an effective amount of betaine to the skin. Further described herein is a method of protecting scalp from biological irritation by sweat or sebum comprising applying an effective amount of betaine to the scalp.

### Brief description of the drawings

Figure 1 shows the results with regard to scalp itchiness received in Example 2.
Figure 2 shows the results with regard to change of general condition received in Example 3.

### Detailed description of the invention

Skin is continuously subjected to various conditions that expose it to biological irritation. According to the present invention biological irritation is induced by a compound secreted by the mammalian body itself, such as sweat or sebum, or an ingredient or a mixture of ingredients thereof.

It has now been discovered that betaine protects skin from biological irritation by sweat or sebum. In the present invention betaine refers to trimethylglycine. The compound is also called trimethylammonioacetate, 1-carboxy-N,N,N-trimethylmeth-aneaminium, inner salt and glycine betaine. It is a naturally occuring quaternary ammonium type compound having the formula

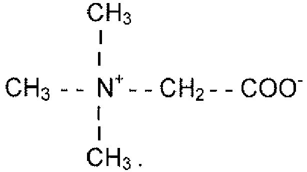

Betaine has a bipolar structure comprising a hydrophilic moiety (COO⁻) and a hydrophobic moiety (N⁺) capable of neutralizing both acid and alkaline solutions.

In its pure form, betaine is a white crystalline compound that is readily soluble in water and lower alcohols. In the present invention betaine can be used, for example, as anhydrous form, or as a monohydrate or a salt. Betaine is commercially available from Finnfeeds Finland Oy as an anhydrous form and also as a monohydrate.

In the present invention, biological irritation refers to the irritation induced by a compound secreted by the mammalian body through the skin. Secretory products, such as sweat and sebum, or an individual ingredient or a mixture of ingredients included in these secretory products may irritate the skin of the body and the scalp especially. Biological irritation is typically temporary by nature. Further, biological irritation is often induced by the environment and/or the circumstances, such as the physical effort and/or exercise. Thus, it is usually also self-inflicted by nature. According to the present invention, biological irritation is not a skin disease or a disease affecting sebaceous glands and/or sweat glands.

In the present invention protecting refers to protecting, preventing and/or alleviating the biological irritation and the symptoms of the irritation, such as itching, stinging and/or burning. The aforementioned symptoms, itching, stinging and burning, can also be symptoms of dry skin. However, the irritation of skin due to dehydration or drying of skin, as such, is not biological irritation according to the present invention.

Skin and scalp are continuously subjected to various conditions that expose it to biological irritation, for example to sweat. In some professions and hobbies the circumstances of the work or the activity expose people to sweating, for example. In addition, there are several professions and also hobbies that require use of protecting equipments, such as, a helmet, a scarf or a cap that by sheltering and covering the skin prevent and/or complicate the evaporation of the secretory products such as the sweat or the sebum, or the individual ingredients of the these secretory products from the skin and/or scalp surface and thus multiply the effect of the biological irritation. Thus, for example the regular use of a helmet, a scarf and/or a cap multiplies the irritating effect of sweat and/or sebum on the scalp. The same applies for the use other protecting equipments and the areas of skin they cover. An area of skin, not necessarily covered by protecting equipment, but otherwise prone to biological irritation, is armpit (axillary fossa). The irritation may be sensed as itching, stinging and/or burning, for example.

Often, for example a scarf or a cap is used under the helmet, which together with hair potentiates the effect of the biological irritation of the scalp.

In addition to sweat, skin generally and scalp specifically excretes sebum. For example exercise, heat, humidity and hormonal action are known to increase the excretion of sebum as well as sweat. Excretion of sebum increases also when scalp is massaged or rubbed and/or hair is brushed.

Sweat contains in addition to water and sodium chloride small amounts of various compounds, such as, potassium chloride, phosphates, amino acids, urea, lactic acid and cresols. Several types of biological waste material and/or excreta are also removed from the body through sweating and excretion of sebum. Sweating and also the secretion of sebum belong to the means of excretion of a mammalian body.

According to the present invention, betaine could be applied to skin before and/or after the occasion or event skin is supposed to be exposed to biological irritation by sweat or sebum. Naturally, the favorable effects of betaine in protecting the skin from biological irritation are achieved when betaine is applied to the skin on continuous basis.

In the present invention a cosmetic or toiletry preparation refers to a skin and/or hair care or a skin and/or hair cleaning/washing product. In general betaine could be applied to the skin in the form of soap, or a conventional emulsion, lotion or cream composition for example. Specifically, when scalp is in question, betaine could be applied to the scalp also in the form of a hair care product, such as but not limited to, an aqueous solution or a shampooing, conditioning or styling formulation. A hair care composition may be in the form of a lotion, mousse, spray, gel, wax or mask. It may be a rinse-off or a leave-on formulation. The cosmetic or toiletry preparations of the invention can be formulated by common methods well known to those skilled in the art.

In one embodiment of the invention betaine is applied to the skin, in general, in a formulation of a skin-care product that is not rinsed from the skin after its application and specifically to the scalp in the form of a leave-in formulation of a hair care product. Leave-on formulation referes herein to a formulation of a skin-care product that is not rinsed from the skin after its application and/or to a leave-on formulation of a hair care product.

Betaine can be included in a preparation in an amount of 0.1 to 50% by weight of the preparation. A cosmetic or toiletry preparation protecting skin from biological irritation according to the present invention contains betaine in an amount of 0.1 to 20% by weight. In one embodiment of the invention, the cosmetic or toiletry preparation contains betaine 0.2 to 10%, preferably 0.5 to 7% by weight and in another embodiment of the invention, the cosmetic or toiletry preparation contains betaine 0.75 to 5% by weight based on the total weight of the preparation. In one embodiment of the invention, betaine is the sole ingredient protecting skin from biological irritation in the cosmetic and/or toiletry preparation. The preparation may also contain one or several ingredients and additives that are commonly used in cosmetic or toiletry preparations. These include, for example, skin care agents, softening agents, astringent agents, refreshing agents, emulsifying agents, moisturizing agents, stabilizing agents, viscosity regulating agents, emollients, thickeners, surfactants, colouring agents, perfumes and the like, as well as alcohols and/or water. The preparations can be formulated by methods known to those skilled in the art.

The following examples illustrate the present invention. The examples are not to be construed to limit the claims in any manner whatsoever.

### Example 1

A shampoo and a leave-on conditioner formulations incorporating betaine in the form of monohydrate were developed. During the development care was taken to restrict the extent of other components in the formulation, so that the properties beneficial with regard to protection of biological irritation can be attributed to the use of betaine, rather that to a plethora of other materials.

**Formulation of a shampoo containing 5% (w/w) betaine:**

| **Trade Name and Supplier** | **INCI Names** | **% w/w** |
|---|---|---|
| Deionised water | Aqua | to 100 |
| Surfac LCX (Surfachem) | Sodium Lauryl Sulphate | 14.0 |
| | Methyl Chloro Isothiazolinone | ∼ |
| | Methyl Isothiazolinone | ∼ |
| | Magnesium Chloride | ∼ |
| | Magnesium Nitrate | ∼ |
| Betafin BP20 (Danisco) | Betaine | 5.0 |
| Cutina AGS (Univar) | Glycol Distearate | 2.0 |
| Laurex CS (S Black) | Cetyl Alcohol | 1.0 |
| Cocamide MEA (S Black) | Cocamide MEA | 1.0 |
| EDTA (S Black) | Tetrasodium EDTA | 0.3 |
| DMDMH (S Black) | DMDM Hydantoin | 0.3 |
| N-Hance 3205 *(Crestchem)* | Guar hydroxypropyltrimonium chloride | 0.2 |
| Methyl Paraben (Clariant) | Methyl Paraben | 0.1 |
| Sodium Chloride (Merck) | Sodium Chloride | ∼ |
| Sodium Hydroxide (Merck) | Sodium Hydroxide | ∼ |
| | Fragrance | 0.2 |

| | | |
|---|---|---|
| ∼ refers to an amount below 0.1 % w/w | | |

**Formulation of a leave-on conditioner containing 2% (w/w) betaine:**

| **INCI Names** | **% w/w** |
|---|---|
| Aqua | 92.45 |
| Betaine | 2.0 |
| PEGIPPG-20/15 Dimethicone | 2.0 |
| Amodimethicone | 1.75 |
| Cetrimonium Chloride | 0.68 |
| Trideceth-12 | 0.55 |
| PEG-40 Hydrogenated Castor Oil | 0.25 |
| Fragrance | 0.2 |
| Disodium EDTA | 0.1 |
| Magnesium Nitrate | ∼ |
| Alpha-isomethyl Ionone | ∼ |
| Benzyl Salicylate | ∼ |
| Methylchloroisothiazolinone | ∼ |
| Linalool | ∼ |
| Geraniol | ∼ |
| Magnesium Chloride | ∼ |
| Citronellol | ∼ |
| Methylisothiazolinone | ∼ |

| | |
|---|---|
| ∼ refers to an amount below 0.1 % w/w | |

In the first trial, a panel of female ringette players tested the shampoo containing 5% (w/w) betaine (the formulation specified above) and found it to alleviate itching and stinging and protect the scalp from irritation.

In the second trial, the panelists tested the leave-on conditioner containing 2% (w/w) betaine (the formulation specified above) and compared it with a leave-on conditioner without betaine. In this trial, all the panelists used same commercially available shampoo. The results received in the second trial are described in Examples 2 and 3.

### Example 2

The effect of betaine on biological irritation was studied with 17 female ringette players wearing a helmet for 1 to 1,5 hours four times a week.

The panelists applied two types of leave-on hair conditioner, one set of products containing betaine and one set of products without betaine. 9 panellists used the betaine products for three weeks and then the products without betaine for three next weeks. For the other 8 panellists the order was reversed, first without betaine, then with betaine. The conditioner with betaine contained it at a level of 2% w/w.

The panellists rated their scalp and hair condition after each week. The condition was rated along several dimensions, e.g. scalp itchiness, hair greasiness, ease of breakage, and general condition. The rating scale was a 9-level subjective scale, number 1 referring to a very poor condition and number 9 referring to a very good condition.

The research questions analyzed were whether betaine affects more favourably the hair condition than the other product. First, each hair condition dimension was compared week by week, betaine vs. non-betaine, using the paired-sample t-test (so there were at most 3.17 = 51 pairs to compare). The scalp itchiness was found to be significantly lower when betaine products were used (p = 0.013, n = 51).

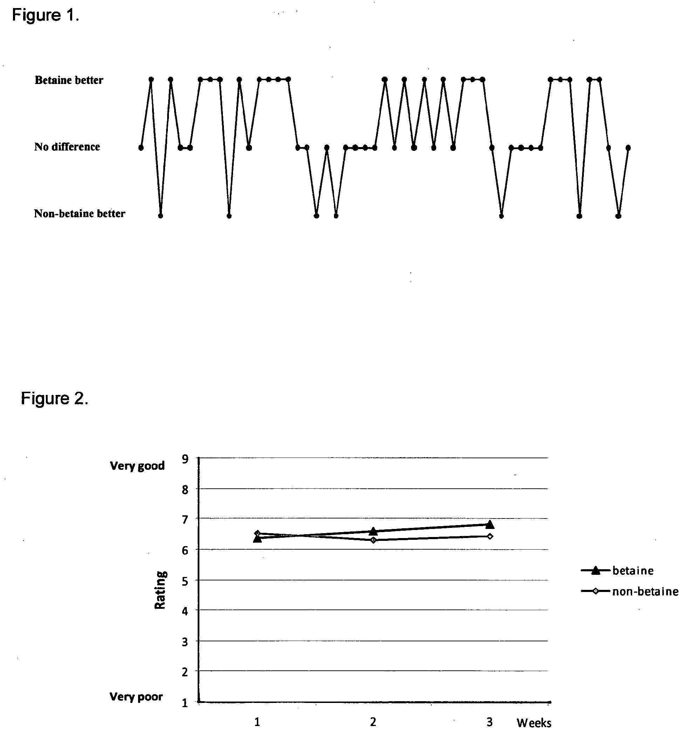

Because the panellists used the scale possibly differently, the results were transformed into a simple dichotomic scale, in order to answer the question "are the betaine products better or worse than the products without betaine". Applying the binomial test it was found that there is a strong statistical difference in scalp itchiness in favour of betaine (p = 0.005, n = 29). Also, the ease of breakage was lower when betaine products were used (p = 0.044, n = 28). Figure 1 shows the results with regard to scalp itchiness.

### Example 3

Another view to compare these two types of leave-on hair conditioner, one set of products containing betaine and one set of products without betaine, is to ask "do the products improve the hair condition when they are used for some time". Therefore, a linear regression analysis was applied to each three-week period. The slopes (how much the hair condition has improved or deteriorated) for betaine products and non-betaine products were then compared. The general condition was in favour of betaine products (p = 0.058, n = 17, paired-sample t-test). The average result of all panelists is shown in Figure 2.

## Claims

1. Betaine for use as a medicament in protecting skin from irritation induced by a compound secreted by a mammalian body through the skin; wherein said compound is selected from sweat, sebum and individual ingredients or mixtures of ingredients included in the secretory products.

2. Betaine for use according to claim 1, **characterized in that** the skin is scalp.

3. Betaine for use according to any one of claims 1 and 2, **characterized in that** the irritation is itching, stinging and/or burning of the skin.

4. Betaine for use according to any one of claims 1 to 3, **characterized in that** betaine is a cosmetic or toiletry preparation applied to the skin in a skin or hair care composition.

5. Betaine for use according to claim 4, **characterized in that** the composition comprises betaine 0.2 to 10%, preferably 0.5 to 7%, more preferably 0.75 to 5 % by weight, based on the total weight of the composition.

6. Betaine for use according to any one of claims 4 or 5, **characterized in that** the composition is formulated into a leave-on formulation.

## Patentansprüche

1. Betain zur Verwendung als Medikament zum Schutz der Haut gegen Reizung, die durch eine von einem Körper eines Säugers durch die Haut ausgeschiedene Verbindung ausgelöst wird, wobei die Verbindung ausgewählt ist aus der Gruppe bestehend aus Schweiß, Talg und individuellen Inhaltsstoffen oder Mischungen von Inhaltsstoffen, die in den sekretorischen Produkten enthalten sind.

2. Betain zur Verwendung nach Anspruch 1, **dadurch gekennzeichnet, dass** die Haut Kopfhaut ist.

3. Betain zur Verwendung nach einem der Ansprüche 1 und 2, **dadurch gekennzeichnet, dass** die Reizung ein Jucken, Stechen oder Brennen der Haut ist.

4. Betain zur Verwendung nach einem der Ansprüche 1 bis 3, **dadurch gekennzeichnet, dass** das Betain ein kosmetisches Präparat oder Toilettenartikel ist, das in einer Zusammensetzung für die Haut- oder Haarpflege auf die Haut aufgetragen wird.

5. Betain zur Verwendung nach Anspruch 4, **dadurch gekennzeichnet, dass** die Zusammensetzung Betain zu 0,2% bis 10 Gew.%, bevorzugt 0,5% bis 7 Gew.%, mehr bevorzugt 0,75% bis 5 Gew.% bezogen auf das Gesamtgewicht der Zusammensetzung aufweist.

6. Betain zur Verwendung nach einem der Ansprüche 4 oder 5, **dadurch gekennzeichnet, dass** die Zusammensetzung in einer "Leave-In" Formulierung konfektioniert wird.

## Revendications

1. Bétaïne pour l'utilisation en tant que médicament dans la protection de la peau contre une irritation induite par un composé sécrété par le corps d'un mammifère à travers la peau; dans laquelle ledit composé est choisi parmi la sueur, le sébum et des ingrédients individuels ou des mélanges d'ingrédients inclus dans les produits de sécrétion.

2. Bétaïne pour l'utilisation selon la revendication 1, **caractérisée en ce que** la peau est un cuir chevelu.

3. Bétaïne pour l'utilisation selon l'une quelconque des revendications 1 et 2, **caractérisée en ce que** l'irritation est une démangeaison, une sensation de piqûre et/ou de brûlure de la peau.

4. Bétaïne pour l'utilisation selon l'une quelconque des revendications 1 à 3, **caractérisée en ce que** la bétaïne est une préparation cosmétique ou de toilette appliquée sur la peau dans une composition pour le soin de la peau ou des cheveux.

5. Bétaïne pour l'utilisation selon la revendication 4, **caractérisée en ce que** la composition comprend la bétaïne à 0,2 à 10%, de préférence 0,5 à 7%, de préférence encore 0,75 à 5% en poids, par rapport au poids total de la composition.

6. Bétaïne pour l'utilisation selon l'une quelconque des revendications 4 ou 5, **caractérisée en ce que** la composition est formulée en une formulation sans rinçage.
